(19) Europäisches Patentamt
European Patent Office
Office européen des brevets

(11) Numéro de publication: **0 286 491 B1**

(12) **FASCICULE DE BREVET EUROPEEN**

(45) Date de publication de fascicule du brevet: **23.12.92**

(51) Int. Cl.5: **C07D 215/40**, C07K 5/06, C07K 5/10, A61K 31/47, A61K 37/02

(21) Numéro de dépôt: **88400683.4**

(22) Date de dépôt: **22.03.88**

Le dossier contient des informations techniques présentées postérieurement au dépôt de la demande et ne figurant pas dans le présent fascicule.

(54) **Dérivés d'aminoquinoléine antimalarique, procédé de préparation et composition pharmaceutique en contenant.**

(30) Priorité: **24.03.87 FR 8704079**

(43) Date de publication de la demande: **12.10.88 Bulletin 88/41**

(45) Mention de la délivrance du brevet: **23.12.92 Bulletin 92/52**

(84) Etats contractants désignés: **AT BE CH DE ES FR GB GR IT LI LU NL SE**

(56) Documents cités:
**EP-A- 0 037 388**

**CHEMICAL ABSTRACTS, vol. 96, no. 7, 15 février 1982, page 24, résumé no. 45906y, Columbus, Ohio, US; A. TROUET et al.: "Development of new derivatives of primaquine by association with lysosomotropic carriers"**

(73) Titulaire: **LA REGION WALLONNE
7 Avenue du Prince de Liège
B-5100 Namur(BE)**

(72) Inventeur: **Falmagne, Jean-Bernard
19, Montagne d'Aisemont
B-1300 Wavre(BE)**
Inventeur: **Pirson, Philippe
25, avenue de Burbure
B-1970 Wesembeek-Oppem(BE)**
Inventeur: **Ouivy, Jacques
22, Cours du Cramignon
B-1348 Louvain-la-Neuve(BE)**
Inventeur: **Trouet, André
29, Predikherenberg
B-3009 Winksele-Herent(BE)**

(74) Mandataire: **Warcoin, Jacques et al
Cabinet Régimbeau 26, avenue Kléber
F-75116 Paris(FR)**

EP 0 286 491 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

CHEMICAL ABSTRACTS, vol. 94, no. 17, 27 avril 1981, page 66, résumé no. 132282u, Columbus, Ohio, US; P. PIRSON et al.: "Antimalarial activity of liposomal primaquine and peptidic derivatives of primaquine"

CHEMICAL ABSTRACTS, vol. 99, no. 17, 24 octobre 1983, page 637, résumé no. 140345k, Columbus, Ohio, US; S. HU et al.: "Synthesis of amino acid and dipeptide derivatives of primaquine"

JOURNAL OF MEDICINAL CHEMISTRY, vol. 29, no. 9, septembre 1986, pages 1765-1769, American Chemical Society; J. HOFSTEENGE et al.: "Carrier-linked primaquine in the chemotherapy of malaria"

## Description

La présente invention s'inscrit dans le domaine du traitement thérapeutique de la malaria humaine, notamment des formes tissulaires.

Les 2,5 milliards de personnes vivant dans les zones endémiques où sévit la malaria rappellent l'échec des efforts développés jusqu'à ce jour pour contrôler cette maladie. 400 millions de personnes vivent dans des régions où aucune mesure prophylactique n'est appliquée. L'Organisation Mondiale de la Santé (O.M.S.) estime qu'un million d'enfants de moins de cinq ans meurent chaque année de la maladie, et ce, dans le seul continent africain.

Il y a quatre espèces de Plasmodies responsables de la maladie chez l'homme. Ils présentent un cycle évolutif similaire impliquant un vecteur, un moustique femelle du genre Anopheles et un hôte, l'homme. Plasmodium falciparum (fièvre tertiaire maligne) est le plus redoutable ; il est responsable des complications mortelles de la malaria cérébrale et est une des premières causes de la mortalité infantile dans les régions endémiques.

Plasmodium vivax et P. ovale (fièvre tertiaire bénigne) sont moins virulents, mais ils produisent des formes latentes dans les hépatocytes, les hypnozoïtes. Ce sont ces formes qui sont responsables des rechutes classiques pouvant persister durant de longues périodes (deux à cinq ans) après une seule infection. P. malariae est moins répandu ; il est responsable de maladies rénales chroniques telles que les néphrites et les syndromes néphrotiques mortels, et peut persister dans le sang pendant plusieurs années.

Plus précisément, la présente invention concerne la mise au point de nouveaux médicaments actifs sur de la malaria humaine, notamment ses formes tissulaires, à savoir de nouveaux dérivés d'aminoquinoléine, notamment de nouveaux dérivés de la primaquine dont la structure est la suivante :

Formule I

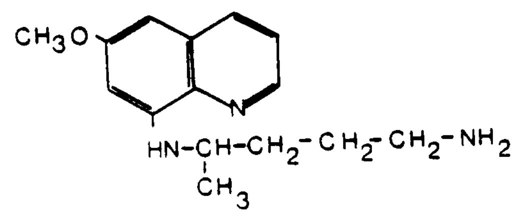

Dans la présente demande de brevet, on entend par aminoquinoléine un dérivé de quinoléine présentant au moins une fonction amine libre, particulièrement les 8-aminoquinoléines parmi lesquelles figurent la primaquine [6-méthoxy-8-(4-amino-1-méthylbutylamino)-quinoléine], mais aussi d'autres 8-aminoquinoléines ayant une fonction amine primaire en fin de chaîne latérale telles que le Quinocide [6-méthoxy-8-(4-aminopentylamino)-quinoléine] ou le SN 3883 [6-méthoxy-8-(4-aminobutylamino)-quinoléine].

Mais, il existe également d'autres dérivés de la primaquine, dont les modifications portent sur le noyau quinoléine (en position 2,4,2-4,5,4-5) et sur la chaîne latérale (4-amino-1-alkylbutylamino) qui sont concernés par la présente invention.

Dans les traitements classiques du paludisme les schizontocides tissulaires essentiellement représentés par les 8-aminoquinoléines (primaquine, quinocide) sont responsables de la prophylaxie causale, à savoir la prévention de l'infection sanguine par la destruction des formes tissulaires (exo-érythrocytaires) primaires à P. falciparum et du traitement radical par l'élimination des formes tissulaires secondaires de P. vivax et P. ovale. La primaquine, une 6-méthoxy-8-(4-amino-1-méthylbutylamino)-quinoléine de formule I, introduite il y a plus de trente ans, est cliniquement efficace sur les formes tissulaires persistantes de P. vivax et P. ovale chez l'homme et est toujours un médicament de choix.

Néanmoins, la toxicité de la primaquine limite son emploi à une application clinique contrôlée. L'effet toxique le plus sérieux est l'hémolyse, particulièrement pour les individus présentant une déficience génétique érythrocytaire, par exemple en glucose-6-phosphate déshydrogénase. La méthaemoglobinurie, les troubles gastro-intestinaux ainsi que certains effets sur le système nerveux central sont également des effets toxiques significatifs de la primaquine et autres 8-amino-quinoléines.

Le nombre de médicaments antimalariques possédant une activité thérapeutique prophylactique causale ou curative radicale est extrêmement limité. La primaquine est le seul médicament pour le traitement curatif radical mais les effets toxiques secondaires en limitent sévèrement l'emploi thérapeutique.

Il est à remarquer que seuls les composés appartenant aux familles des 8-aminoquinoléines sont susceptibles de produire une guérison radicale.

Le but de la présente invention est donc de proposer de nouveaux dérivés de quinoléine antimalarique, notamment de nouveaux dérivés de 8-aminoquinoléines possédant un index thérapeutique plus favorable.

Autrement dit, le but de la présente invention est de réduire la toxicité intrinsèque desdites quinoléines antimalariques telles que la primaquine et d'augmenter leur activité, notamment pour la primaquine, sur les formes tissulaires hépatiques.

Pour ce faire, la présente invention a pour objet de nouveaux dérivés d'aminoquinoléine antimalarique, notamment de nouveaux dérivés de la primaquine, dont la caractéristique essentielle est qu'ils sont représentés schématiquement par la formule PQ-X, dans laquelle

- PQ est une 8-aminoquinoléine antimalarique et X est choisi parmi la L-alanine, la L-phénylalanine, l'acide L-glutamique.
- la liaison PQ-x étant une liaison covalente peptidique entre le groupement amine libre de PQ et le groupement carboxylique terminal de X.

La primaquine agit comme un oxydant et destabilise la membrane des globules rouges provoquant une hémolyse qui est à l'origine de sa toxicité principale.

L'adjonction d'un amino-acide, conformément à la présente invention, réduit la pénétration de la primaquine dans les globules rouges en raison de l'encombrement stérique.

En outre, de manière tout à fait surprenante, l'activité de ces nouveaux dérivés est apparue, comme on le verra plus loin, beaucoup plus importante.

L'article du Journal of Medicinal Chemistry 1986, vol 29, p.1765, décrit la préparation de cystéinylprimaquine. L'article du Bulletin of the W.H.O. 59(3), 459-488, 1981 décrit un dérivé Leucyl-Primaquine.

On notera que la L-leucine liée à la primaquine a été proposée dans la demande EP 37 388 compte-tenu de son intérêt en tant que linker ou bras et donc dans le cadre de conjugué vecteur-bras-drogue. En effet, la L-leucyl-primaquine est stable dans le sérum et régénère le principe actif libre une fois soumis à la digestion lyposomiale. Toutefois, l'index thérapeutique des dérivés de PQ avec la L-leucine adjacente non conjuguée s'est avérée moins intéressants que ceux des dérivés qui font l'objet de la présente invention comme il sera vu ci-après.

Pour la primaquine, des activités thérapeutiques particulièrement remarquables sont obtenues en effet pour la L-lysyl-PQ, la L-phénylalanyl-PQ, la L-alanyl-PQ et la L-glutamyl-PQ.

Une réduction de toxicité importante est obtenue pour la L-glutamyl-PQ et la L-alanyl-PQ.

Les deux effets combinés (activité et toxicité) permettent une augmentation importante de l'index thérapeutique dans le cas des dérivés L-glutamyl-PQ (16,1), L-alanyl-PQ (7,5) et L-phénylalanyl-PQ (5,3).

Dans cette demande de brevet, on entendra par dérivés aminoacyles ou dérivés aminoacides d'aminoquinoléine le produit obtenu par greffage d'un aminoacide sur ladite aminoquinoléine, conformément à la présente invention.

Les produits de base PQ ainsi que les produits obtenus PQ-X peuvent se présenter sous forme de leurs sels d'addition avec des acides.

La présente invention a également pour objet un procédé de préparation de ces nouveaux dérivés d'aminoquinoléine antimalarique, notamment des nouveaux dérivés de primaquine.

Dans la caractéristique essentielle du procédé selon la présente invention, on fait réagir par condensation un aminoacide sous forme acide, dont la fonction amine est protégée, avec l'aminoquinoléine ou un de ses sels portant donc une fonction amine libre, en présence d'un agent de condensation.

Les groupements protecteurs des la fonction amine sont des groupements classiques, mais de préférence on utilise le tertiobutyloxycarbonyle.

La fonction acide de l'aminoacide peut aussi être activée par exemple sous forme d'ester avec le N-hydroxysuccinimide.

En général, l'agent de condensation est un carbodiimide comme le dicyclohexylcarbodiimide.

Selon un mode de réalisation particulier du procédé, on part d'un sel d'aminoquinoléine, par exemple du diphosphate de primaquine et de l'acide aminé dont la fonction aminée est protégée par exemple par un groupe terbutyloxycarbonyle et on effectue les étapes suivantes :

a) on fait réagir de la N-hydroxysuccinimide ou tout autre groupe activant la fonction acide de l'acide aminé, sur le dérivé protégé de l'acide aminé, par exemple sur le dérivé N-terbutyloxycarbonyle de l'acide aminé,

b) on fait réagir l'aminoquinoléine, telle que la primaquine, libérée à partir de son sel par exemple par une solution d'ammoniaque, on la fait réagir donc avec par exemple l'ester N-hydroxysuccinimide du dérivé protégé de l'acide aminé,

c) le produit brut ainsi obtenu est purifié par chromatographie sur silicagel,

d) le groupement protecteur, tel que le terbutyloxycarbonyle du composé obtenu est ensuite clivé en présence d'acide, par exemple l'acide trifluoroacétique pour donner le dérivé PQ-X sous forme de sel, par exemple trifluoroacétate.

Ce groupement peut en effet être éliminé dans des conditions qui ne détruisent pas la liaison

peptidique formée.

Selon une autre caractéristique du procédé, le produit obtenu à l'étape d) est purifié par chromatographie sur support phase inverse.

Cette purification présente un intérêt industriel avec la possibilité de mise en série de colonnes.

Plus précisément, on utilise par exemple comme éluant un mélange d'acétonitrile dans l'eau contenant de l'acide, par exemple trifluoroacétique.

Selon une autre caractéristique particulière du procédé, le contre anion trifluoroacétate est échangé en chlorhydrate.

Dans le but d'inhiber les réactions d'oxydation, une autre caractéristique particulière du procédé prévoit que la solution aqueuse contenant le sel de chlorhydrate de dérivés selon la présente invention est traitée par du bisulfite de sodium.

La présente invention a également pour objet les compositions pharmaceutiques contenant à titre de principe actif les nouveaux dérivés selon la présente invention ou leurs sels pharmaceutiquement acceptables.

D'autres caractéristiques et avantages de l'invention apparaîtront à la lumière des exemples qui vont suivre.

Exemples I à VII : SYNTHESE DES DERIVES AMINOACYLES DE LA PRIMAQUINE. (Pq = Primaquine)

Les dérivés aminoacyles de la primaquine sont obtenus en deux étapes au départ de la primaquine diphosphate et de l'acide aminé dont la fonction aminée est protégée par un groupe N-terbutyloxycarbonyle (N-TBoc). La première étape comprend la réaction de la primaquine base avec l'ester N-hydroxysuccinimide du dérivé N-TBoc de l'acide aminé.

Le produit brut ainsi obtenu est alors purifié par chromatographie sur silicagel. On isole avec de bons rendements (± 70 %) du produit obtenu sous forme d'un solide ± cristallin assez hygroscopique.

Le groupe TBoc est ensuite clivé en présence d'acide trifluoroacétique. Le dérivé aminoacyle de la primaquine est obtenu sous forme trifluoroacétate (probablement un sel ditrifluoroacétate).

Dans le cas où une purification ultérieure est nécessaire, on effectue une colonne de chromatographie à phase inverse et le produit est élué par le mélange acétonitrile/$H_2O$.

Le dérivé aminoacyle de la primaquine sous sa forme base libre peut être obtenu en neutralisant la solution aqueuse contenant le sel de trifluoroacétate d'aminoacyle-Pq par $NH_4OH$ jusqu'à pH ± 8 suivi d'une extraction vigoureuse par le dichlorométhane. On obtient, après évaporation du $CH_2Cl_2$, une huile verdâtre très difficilement manipulable qui peut être purifiée ultérieurement par chromatographie sur colonne de silicagel élué avec un mélange $CH_2Cl_2$ - EtOH - $NH_4OH$ (120-20-1).

Dans la méthode de purification des dérivés aminoacyles de la primaquine sous leur forme trifluoroacétate utilisant la technique de chromatographie sur colonne phase inverse, la colonne utilisée est une colonne Merck lobar 8. Le support est une silice greffée par des résidus en $C_8$. Afin d'éviter un processus d'oxydation des dérivés sur la colonne, les solvants d'élution sont soigneusement dégazés. Pour la même raison, on peut envisager d'autres types de silice greffée comme phase stationnaire, notamment complètement déminéralisés. Le produit brut dissous en milieu acide aqueux est adsorbé en tête de colonne ; on procède ensuite à une étape de désalage ($H_2O$-$H^+$) avant d'éluer le produit en utilisant un gradient de $CH_3CN$ dans $H_2O/H^+$. L'éluat est fractionné. Les fractions contenant le produit (fractions colorées jaune orange) sont analysées par HPLC. Celles contenant le produit avec une pureté supérieure à 98 % sont rassemblées et lyophilisées. On obtient une poudre jaune orange vive. Le contre anion trifluoroacétate est alors échangé en chlorhydrate en additionnant à une solution aqueuse du sel de trifluoroacétate une quantité d'HCl aqueuse correspondant approximativement à deux équivalents d'acide calculé par rapport au poids de sel de trifluoroacétate à échanger. La solution obtenue (pH 2,8-3) est lyophilisée à l'abri de la lumière (2 lyophilisations). Le produit obtenu se présente sous forme d'une poudre orange vive très hygroscopique. Même stocké sous atmosphère inerte (argon), le produit se dégrade assez rapidement au cours du temps. Il présente également une stabilité limitée en solution : milieu physiologique ou solution aqueuse à 0° et à l'abri de la lumière.

Le noyau quinoléique est une cible particulièrement sensible pour toute réaction d'oxydation. Celle-ci explique partiellement l'instabilité observée. Dans le but d'inhiber ces réactions d'oxydation, nous avons traité la solution aqueuse contenant le sel de trifluoroacétate par du bisulfite de sodium (0,1 %).

La proportion relative $CH_3CN$-$H_2O/H^+$ varie suivant la nature de l'acide aminé greffé sur la primaquine.

Exemple I : Synthèse de la N-1-L-alanyl, N-4-(méthoxy-6-quinolinyl-8) pentanediamine-1,4.

A 1 g (5,9 mmoles) de N-tBoc-alanine, dissous dans 10 ml de diglyme, maintenu à 0 ° C, on ajoute 0,61 g (5,3 mmoles) de la N-hydroxysuccinimide et 1,09 g (5,3 mmoles) de dicyclohexylcarbodiimide. Après 4 heures de réaction, on ajoute 1,37 g (5,3 mmoles) de primaquine base. Cette dernière est libérée de la primaquine diphosphate par l'action d'une solution d'ammoniaque 25 %. Après 17 heures, la dicyclohexylurée est filtrée et le solvant chassé sous vide. L'huile brune obtenue est dissoute dans le $CH_2Cl_2$ et lavée à l'eau. La phase organique est séchée sur sulfate de sodium, filtrée et évaporée. Le produit est purifié sur gel de silice en utilisant comme éluant un mélange dichlorométhane-méthanol. Les fractions pures sont rassemblées et l'éluat évaporé conduisant à 1,5 g (66 %) d'un produit plus ou moins cristallin, assez hygroscopique.

RMN : ($CDCl_3$ - TMS) :
1.2 - 1.7 (m, 19 H) ; 3.3 (m, 2 h) ; 3.64 (m, 1 H) ; 3.9 (s, 3 H) ; 4.07 (m, 1 H) ; 5 (m, 1 H) ; 6 (m, 1 H) ; 6.2 (m, 1 H) ; 5.26 (d, 1 H) ; 6.33 (D, 1 H) ; 7.3 (dd, 1 H) ; 7.9 (dd, 1 H) ; 8.5 (dd, 1 H).

Le composé obtenu précédemment est dissous dans 15 ml d'un mélange 1:1 de dichlorométhane et d'acide trifluoroacétique. Le mélange est agité 30 minutes à température ambiante. Les solvants sont évaporés sous vide et le résidu est repris dans de l'eau et lavé plusieurs fois au diisopropyléther.

Si nécessaire, le produit est purifié par chromatographie sur phase inverse (Merck lobar 8) en utilisant comme éluant un mélange de 25 % d'acétonitrile dans de l'eau contenant 1 % d'acide trifluoroacétique. Les fractions pures (HPLC) sont rassemblées, l'acétonitrile chassé sous vide. Après 2 cycles de lyophilisation, on obtient 1,6 g (80 %) d'une poudre orange vive hygroscopique.

RMN : ($D_2O$-ref externe) :
1.65 - 1.50 (m, 6 H) ; 1.88 (m, 4 H) ; 3.45 (m, 2 H); 5 (m, 1 H) ; 4.17 (m, 4 h) ; 7.08 (s, 1 H) ; 7.13 (d, 1 H) ; 7.93 (dd, 1 H) ;8.65 (d, 1 H) ; 8.88 (d, 1 H).

Exemple II : Synthèse de la N-1-D-alanyl, N-4-(méthoxy-6-quinolinyl-8)pentanediamine- 1,4.

En suivant le même mode opératoire que celui décrit à l'exemple I, on isole le composé du titre au départ de la primaquine et de N-tBoc-D-alanine et après déprotection de la fonction amine à l'aide de l'acide trifluoroacétique (52 %).

RMN : ($CD_3OH$ - ref ext.) :
1.40 (d, 3H) ; 1,54 (dd, 3H) ; 1.80 (m, 4H) ; 3.80 (m, 1H) ; 3,94 (q, 1H) ; 4 (s, 3H) ; 6.6 (s, 2H) ; 7,35 (dd, 1H) ; 8,37 (m, 1H) ; 8.68 (dd, 1H).

Exemple III : Synthèse de la N-1-L-leucyl, N-4-(méthoxy-6-quinolinyl-8)pentanediamine- 1,4.

En suivant le même mode opératoire que celui décrit à l'exemple I, on isole le composé du titre au départ de la primaquine et de N-tBoc-L-leucine et après déprotection de la fonction amine à l'aide de l'acide trifluoroacétique (70 %).

RMN : ($H_2O$ ref externe) :
0.71 - 0.81 (m, 6 H) ; 1.28 (d, 3 H) ; 1.4 - 1.7 (m, 7 H) ; 3 - 3.2 (m, 1 H) ; 3.26 - 3.5 (m, 1 H) ; 3.6 - 4 (m, 5 H) ; 6.8 - 6.9 (m, 2 H) ; 7.65 -7.72 (m, 1 H) ; 8.52 (d, 1 H) ; 8.63 (d, 1 H).

Exemple IV : Synthèse de la N-1-D-leucyl, N-4-(méthoxy-6-quinolinyl-8) pentanediamine- 1,4.

En suivant le même mode opératoire que celui décrit à l'exemple I, on isole le composé du titre au départ de la primaquine et de N-tBoc-D-leucine et après déprotection de la fonction amine à l'aide de l'acide trifluoroacétique (78 %).

RMN : ($CD_3OD$ ref ext.) :
1.05 (m, 6H) ; 1.4 (d, 3H) ; 1.77 (m, 6H) ; 3.36 (m, 2H) ; 3.7 (m, 2H) ; 4 (s, 3H) ; 6,66 (s, 2H) ; 7,67 (dd, 1H) ; 8.46 (ddd, 1H) ; 8.72 (dd, 1H).

Exemple V : Synthèse de la N-1-L-phénylalanyl , N-4(methoxy-6-quinolinyl-8) pentanediamine- 1,4.

En suivant le même mode opératoire que celui décrit à l'exemple I, on isole le composé du titre au départ de la primaquine et de N-tBoc-L-phénylalanine et après déprotection de la fonction amine à l'aide de l'acide trifluoroacétique (79 %).

RMN : ($CD_3OD$ - ref ext.) :
1.35 (dd, 3H) ; 1.62 (m, 4H) ; 3.16 (m, 2H) ; 3.40 (m, 2H) ; 3.71 (m, 1H) ; 3.92 (d, 3H) ; 4.03 (dt, 1H) ; 6.38 (m, 1H) ; 6.53 (m, 1H) ; 7.32 (m, 5H) ; 7.46 (dd, 1H) ; 8.13 (m, 1H) ; 8.59 (dd, 1H).

Exemple VI : Synthèse de la N-1-L-lysyl, N-4-(méthoxy-6-quinolinyl-8 pentanediamine- 1,4.

En suivant le même mode opératoire que celui décrit à l'exemple I, on isole le composé du titre au départ de la primaquine et de N-tBoc-L-lysine et après déprotection de la fonction amine à l'aide de l'acide trifluoroacétique.

Exemple VII : Synthèse de la N-1-L-glutamyl, N-4-(méthoxy-6-quinolinyl-8) pentadiamine-1,4.

En suivant le même mode opératoire que celui décrit à l'exemple I, on isole le composé du titre au départ de la primaquine et de l'acide N-tBoc glutamique et après déprotection de la fonction amine à l'aide de l'acide trifluoroacétique .

Exemple VIII : Synthèse de la N-1-L-Alanyl-L-Leucyl, N-4-(Méthoxy-6-quinolinyl-8) pentanediamine-1,4.

A 686 mg (3 mmoles) de N-t-Boc-Leucine dissous dans 7 ml de diméthylformamide maintenu à 0 ° C, on ajoute 346 mg (3 mmoles) de la N-hydroxy succinimide et 6,3 mg (3 mmoles) de dicyclohexylcarbodiimide.

La réaction est ensuite maintenue pendant 24 heures à 4 ° C.

D'autre part, on prépare une solution de 1,5 g (2,7 mmoles) du sel de trifluoroacétate de la L-alanylprimaquine dans 7 ml de dichlorométhane a laquelle on ajoute 593 µl (5,4 mmoles) de N-méthylmorpholine.

Après 24 heures de réaction à 20 ° C, on évapore les solvants. L'huile brune ainsi obtenue est dissoute dans de l'acétate d'éthyle. La phase organique est ensuite lavée 5 fois avec de l'eau. Les phases aqueuses sont rassemblées et extraites une fois à l'acétate d'éthyle. Les phases organiques sont rassemblées, séchées sur sulfate de sodium, filtrées et évaporées. L'huile brune obtenue est dissoute dans le minimum d'acétate d'éthyle et purifiée sur une colonne de silicagel éluée avec 400 ml du mélange acétate d'éthyle-hexane 50-50 puis par 400 ml d'acétate d'éthyle pur. Les fractions pures sont rassemblées et évaporées à sec. On obtient ainsi 1 g de N-t-Boc-L-Leu-L-Ala-primaquine.

Ce produit est directement engagé dans la réaction de déprotection soit le mélange de 5 ml de dichlorométhane et de 5 ml d'acide trifluoroacétique pendant 30 minutes à température ambiante.

Les solvants sont évaporés sous vide et le résidu repris dans de l'eau est extrait avec le mélange 1:1:1 de diéthyléther, diisopropyléther et hexane. La phase aqueuse est ensuite lyophilisée. Après deux lyophilisations, on obtient 1,2 g (66 %) de dipeptide.

Exemples IX à XIII : MODELES THERAPEUTIQUES

Le modèle de la malaria du rongeur, Plasmodium berghei-Anopheles stephensi-souris, mis au point par MOST, H., HERMAN, R. & SCHOENFELD, C. (1967) Am. J. Trop. Med. Hyg. 16, 572-575, permet de déterminer l'activité prophylactique causale (schizonticide tissulaire) de nouveaux composés thérapeutiques. La procédure utilisée est dérivée de celle de MOST, H. & MONTUORI, W.A. (1975) Am. J. Trop. Med. Hyg. 24, 179-182. Il s'agit du système P. berghei berghei (ANKA)- Anopheles stephensi-souris Swiss OF1. La souche ANKA de P. b. berghei est entretenue cycliquement sur Anopheles stephensi. La souche de souris Swiss OF1 est particulièrement sensible à P. b. berghei qui produit une infection toujours mortelle.

Exemple IX :Isolement des sporozoïtes des moustiques et infection expérimentale

Les moustiques femelles (80 à 100 individus par préparation) sont débarassés de leurs pattes, ailes et abdomen, puis homogénéisés à 4 ° C à l'aide d'un potter en verre, dans du milieu M 199 (GIBCO) contenant 10 % de sérum foetal de veau. La suspension de sporozoïtes est ensuite purifiée des débris du moustique par deux centrifugations successives ( 2 min. à 50 g et 5 min. à 200 g) et le surnageant recueilli est conservé à 4 ° C. Après comptage dans une chambre de Petroff-Hausser, le nombre de parasites est ajusté par dilution avec le même milieu froid, à la densité de 2 x $10^5$ sporozoïtes/ml. L'ensemble de la procédure est toujours effectuée dans un maximum de 35 minutes et permet de préserver la viabilité et l'infectivité des sporozoïtes.

Un volume de 0,5 ml de la suspension contenant $10^5$ sporozoïtes est injectée i.v. aux souris Swiss OF1 femelles de 18 à 22 g utilisées pour les tests thérapeutiques.

Exemple X : Traitements chimiothérapeutiques

Des groupes de 20 à 25 souris infectées comme décrit ci-dessus sont traités en une seule injection intraveineuse de primaquine diphosphate (Pq) ou des dérivés monoacyls et aminoacides de la primaquine sous formes dichlorhydrates (X-Pq) trois heures après l'inoculation des sporozoïtes. Le volume injecté est ajusté en fonction du poids de la souris, à raison de 0,5 ml pour un poids de 25 g. Les composés sont dilués dans du tampon phosphate salin (PBS) pH 7.0 et le pH est ajusté à 7.0. Les souris infectées mais non traitées (5 par groupe) reçoivent le tampon seul et servent à contrôler l'infectivité des sporozoïtes.

Exemple XI :Paramètres utilisés pour évaluer l'efficacité de la primaquine

Les souris expérimentales sont gardées durant une période de 50 jours. Le développement de la parasitémie est suivi en examinant quotidiennement des frottis de sang, colorés 60 min. au Giemsa à 5 % dans du tampon Sörensen à pH 7,2, à partir du 5ème jour et ce jusqu'au 15ème jour. Le nombre d'animaux infectés peut ainsi être déterminé. Ces résultats permettent d'établir la survie à long terme (LTS, le nombre de souris survivantes non parasitées sur le nombre de souris infectées et traitées).

L'activité prophylactique causale ($CPD_{50}$) est obtenue par l'analyse des logits de la réponse (exprimée en % de LTS) en fonction de la dose (mg Pq/kg).

L'effet résiduel éventuel des médicaments sur les premières formes érythrocytaires (effet schizontocide érythrocytaire) est déterminé par la méthode de GREGORY, K.G. & PETERS, W. (1970) Ann. Trop. Med. Parasitol. 64, 15-24, basée sur le test des 2 % de Warhurst.

Exemple XII : Etude de la toxicité des composés

La toxicité aiguë en fonction de la dose unique administrée est déterminée sur des souris de 18 à 22 g. Une série de dilutions de chaque composé est injectée par voie intraveineuse à des groupes de 10 souris.

La dose léthale pour 50 % des souris non infectées ($LD_{50}$) est déterminée par régression linéaire des logits du pourcentage des animaux survivants, en fonction du logarithme de la dose injectée (en mg Pq diphosphate équivalent/kg) (Logit Weighted Transformation ; BERKSON, J. (1953) J. Am. Stat. Assoc. 48, 565-599.

Exemple XIII: Activité thérapeutique et toxicologique in vivo de la primaquine et de ses dérivés aminoacides

Les animaux infectés à l'aide de sporozoïtes de P. berghei sont traités trois heures après avec une seule injection des composés par voie intraveineuse. Ce test permet de définir l'activité prophylactique causale des composés ou la dose permettant de guérir 50 % des animaux infectés ($CPD_{50}$). La dose est toujours exprimée en mg de Pq diphosphate par kg.

Les résultats de la toxicité de la primaquine et de ses dérivés aminoacides et dipeptidiques sont présentés au tableau 1. Les résultats de l'activité prophylactique causale (+ 3 heures) de la primaquine et des différents dérivés sont présentés aux tableaux 3 à 8 et résumés au tableau 2.

Pour la primaquine (tableau 3), la dose effective minimale ($E_M$) est de 10,4 mg Pq/kg. L'efficacité prophylactique causale ($CPD_{50}$) de la Pq est de 18,7 mg Pq/kg. Du fait de la toxicité de la Pq dès 25 mg/kg, il n'est pas possible d'obtenir un traitement curatif. L'activité thérapeutique obtenue à 25 mg/kg est de 75 %. Les résultats relatifs à la toxicité de la Pq sont également au tableau 1. La dose maximale tolérée (MTD) est de 25,7 mg Pq/kg et la dose léthale 50 % ($LD_{50}$) est de 31 mg Pq/kg. L'index thérapeutique de la Pq, représentant le rapport de la $LD_{50}$ et du $CPD_{50}$, est de 1,7.

Le premier dérivé, la L-leucyl-Pq (tableau 2), se montre un peu plus actif que la Pq. La dose minimale effective et le $CPD_{50}$ sont respectivement de 7,6 mg Pq/kg et de 16,2 Pq/kg. A la dose de 30 mg Pq/kg, un traitement curatif total (100 %) est obtenu. L'index Pq n'est que de 1,2.

Tableau I : Toxicité et activité comparées de la Primaquine et de ses dérivés aminoacides.

| Dérivés quinoléiniques [a] | MTD [b] | $LD_{50}$ [c] | $CPD_{50}$ [d] | TI [e] |
|---|---|---|---|---|
| Pq | 24,6 | 31,3 | 18,7 | 1,7 |
| L-leu-Pq | 33,7 | 48,0 | 16,2 | 2,8 |
| L-ala-Pq | 34,6 | 47,5 | 6,3 | 7,5 |
| D-ala-Pq | 33,8 | 42,9 | 14,5 | 3,0 |
| L-glu-Pq | 124,0 | 136,0 | 8,5 | 16,1 |
| L-gln-Pq | - | - | 10,0 | - |
| L-lys-Pq | 8,2 | 10,1 | 3,9 | 2,6 |
| L-phe-Pq | 20,0 | 32,2 | 6,1 | 5,3 |
| L-leu-ala-Pq | 38,8 | 51,2 | 13,9 | 3,7 |

[a] : les dérivés sont administrés par vote i.v. trois heures après l'inoculation des sporozoïtes. Les concentrations sont toujours exprimées en mg Pq diphosphate/kg.

[b] : la dose maximale tolérée est définie code la dose la plus élevée induisant une perte de poids inférieure ou égale & 5 % du poids initial pendant une période de cinq jours suivant l'injection i.v.

[c] : dose léthale tuant 50 % des souris en une seule injection i.v.

[d] : dose prophylactique causale permettant de guérir 50 % des souris injectées.

[e] : index thérapeutique défini par le rapport $LD_{50}/CPD_{50}$.

Tableau 2 : Activité prophylactique causale de la Primaquine diphosphate et de ses dérivés.

| Composés[a] | $CPD_{50}$[b] | MED[c] | $DE_{100}$[c] | Index Pq[d] | TI[e] |
|---|---|---|---|---|---|
| Primaquine | 18,7 | 10,4 | TOX(25) | 1,0 | 1,7 |
| L-leu-PQ | 16,2 | 7,6 | 30,7 | 1,2 | 2,8 |
| L-ala-PQ | 6,3 | 4,0 | 9,9 | 3,0 | 7,5 |
| D-ala-PQ | 14,5 | 3,6 | - | 1,3 | - |
| L-Lys-PQ | 3,9 | 1,7 | 8,8 | 4,8 | 2,6 |
| L-phe-PQ | 6,1 | 1,8 | 21,3 | 3,1 | 5,3 |
| L-glu-Pq | 8,5 | 4,0 | 17,7 | 2,2 | 16,1 |
| L-gln-PQ | 10,0 | 4,5 | 22,4 | 1,9 | - |

[a] : Les composés sont administrés i.v. 3 heures après l'inoculation des sporozoïtes de P.berghei. La dose est exprimée en mg PQ diphosphate équivalent/kg.

[b] : Dose prophylactique causale guérissant 50% des animaux infectés.

[c] : Dose minimale effective (MED) et dose totalement curative (DE 100).

[d] : L'index PQ est défini par le rapport : $CPD_{50}$ de la PQ/$CPD_{50}$ du composé.

[e] : L'index thérapeutique est le rapport entre le $LD_{50}$ et la $CPD_{50}$.

Tableau 3 : Activité prophylactique causale de la Primaquine diphosphate

| Dose[a] (mg/kg) | LTS/N[b] | LTS (%) | $CPD_{50}$[c] | TI[d] |
|---|---|---|---|---|
| Contrôles | 0/30 | 0 | - | |
| 10 | 0/12 | 0 | | |
| 15 | 6/22 | 27 | 18,65 | 1,7 |
| 20 | 15/26 | 58 | | |
| 25[e] | 6/8 | 75 | | |

[a] : La Pq est administrée i.v. 3 heures après l'inoculation des sporozoïtes de P. berghei. La dose est exprimée en mg Pq diphosphate/kg.

[b] : Survivants à long terme au jour 50 sur le nombre de souris infectées et traitées.

[c] : Dose prophylactique causale guérissant 50% des animaux.

[d] : L'index thérapeutique est le rapport entre la $LD_{50}$ et la $CPD_{50}$.

[e] : Toxicité à l'injection.

La L-alanyl-Pq (tableau 4), possède une activité thérapeutique nettement plus grande que la Pq et ses dérivés leu-Pq. La dose minimale effective est de 4 mg Pq/kg tandis que le $CPD_{50}$ est de 6,3 mg Pq/kg. La dose de 9,9 mg Pq/kg est totalement curative. L'index Pq est de 3,0 et l'index thérapeutique de la L-alanyl-Pq est de 7,5.

Ce dérivé est également moins toxique que la primaquine (tableau I).

Ainsi, pour la L-alanine, on obtient des résultats particulièrement intéressants, notamment une activité antimalarique presque quatre fois supérieure à celle de la primaquine base.

La D-alanine est intéressante aussi en ce qu'on obtient avec elle une très faible toxicité. En effet, la liaison avec le produit D est moins réversible. Ainsi, les métabolites pénètrent en moins grande quantité dans les globules rouges.

Tableau 4 : Activité prophylactique causale de la L-alanyl-Primaquine

| Dérivés quinoléiniques[a] | Dose (mg/kg) | LTS/N[b] | LTS(%) | $CPD_{50}$[c] | Index Pq[d] |
|---|---|---|---|---|---|
| Contrôles | - | 0/25 | 0 | | |
| L-ala-Pq | 4,0 | 0/15 | 0 | | |
| | 4,6 | 4/20 | 20 | | |
| | 5,4 | 4/15 | 27 | | |
| | 6,0 | 6/15 | 40 | 6,3 | 3,0 |
| | 6,8 | 6/10 | 60 | | |
| | 7,3 | 8/10 | 80 | | |
| | 10,4 | 5/5 | 100 | | |

[a] : Les deux dérivés de la primaquine sont administrés par voie i.v. 3 heures après l'inoculatin des sporozoïtes. Les concentrations sont également exprimées en équivalent Pq diphosphate/kg.

[b] : Survivants à long terme au jour 50 sur le nombre de souris infectées et traitées.

[c] : Dose prophylactique causale guérissant 50% des animaux infectés.

[d] : L'index Pq est le rapport entre la $CPD_{50}$ de la Pq et la $CPD_{50}$ du dérivé.

L'activité prophylactique causale de la L-phenylalanyl-Pq est présentée au tableau 5. La $CPD_{50}$ de ce dérivé est de 6,1 mg Pq/kg donnant un index Pq de 3,1. La dose minimale effective est de 1,8 mg Pq/kg. Ce dérivé est totalement curatif à la dose de 21,3 mg Pq/kg. La toxicité de ce composé est voisine de celle de la Pq. L'index thérapeutique est de 5,3.

La L-lysyl-Pq est également nettement plus active que la Pq avec une $CPD_{50}$ de 3,9 mg Pq/kg et un index Pq de 4,8 (tableau 6). La dose minimale effective est de 1,7 mg Pq/kg. Néanmoins, ce composé se montre toxique à plus forte dose. A la dose minimale toxique de 8,2 mg Pq/kg, le composé est totalement curatif. L'index thérapeutique est supérieur à celui de la Pq (2,6).

L'addition de l'acide L-glutamique ou de la L-glutamine permet également d'accroître l'efficacité de la primaquine (tableau 7). Les $CPD_{50}$ sont respectivement de 8,5 mg Pq/kg et 10 mg Pq/kg et l'index Pq est successivement de 2,2 et 1,9. Le dérivé L-glutamyl-Pq apparaît comme le moins toxique. La MTD est de 124 mg Pq/kg (5x > à la MTD de la Pq) et la $LD_{50}$ est de 136 mg Pq/kg (4x > à la $LD_{50}$ de la Pq).

Les trois derniers dérivés acides aminés de la primaquine, la succinyl-L-alanyl-Pq, la succinyl-L-phenylalanyl-Pq et la glutarryl-Pq sont nettement moins actifs que la Pq.

Tableau 5 : Activité prophylactique causale de la L-phenylalanyl-primaquine

| Dérivés quinoléiniques[a] | Dose (mg/kg) | LTS/N[b] | LTS (%) | $CPD_{50}$[c] | Index[d] Pq |
|---|---|---|---|---|---|
| Contrôles | - | 0/20 | 0 | - | - |
| | 1,6 | 0/5 | 0 | | |
| L-phe-Pq | 3,4 | 5/20 | 25 | | |
| | 6,7 | 12/20 | 60 | 6,1 | 3,1 |
| | 10,1 | 13/20 | 65 | | |
| | 12,9 | 10/12 | 80 | | |
| | 13,7 | 13/15 | 87 | | |

[a] : Le dérivé de la Primaquine est administré par voie i.v. 3 heures après l'inoculation des sporozoïtes. Les concentrations sont également exprimées en équivalent Pq diphosphate/kg.

[b] : Survivants à long terme au jour 50 sur le nombre de souris infectées et traitées.

[c] : Dose prophylactique causale guérissant 50% des animaux infectés.

[d] : L'index Pq est le rapport entre la $CPD_{50}$ de la Pq et la $CPD_{50}$ du dérivé.

Tableau 6 : Activité prophylactique causale de le L-lysyl-primaquine

| Dérivés quinoléiniques[a] | Dose (mg/kg) | LTS/N[b] | LTS (%) | $CPD_{50}$[c] | Index[d] Pq |
|---|---|---|---|---|---|
| Contrôles | - | 0/20 | 0 | - | - |
| | 1,9 | 2/15 | 13 | | |
| | 3,4 | 6/21 | 29 | | |
| L-lys-Pq | 5,6 | 7/10 | 70 | | |
| | 6,4 | 18/22 | 82 | 3,9 | 4,8 |
| | 8,0 | 5/5 | 100 | | |
| | 9,2 | 7/12 | TOX 29 % | | |
| | 12,3 | 0/5 | TOX 100 % | | |

[a] : Le dérivé de la Primaquine est administré par voie i.v. 3 heures après l'inoculation des sporozoïtes. Les concentrations sont également exprimées en équivalent Pq diphosphate/kg.
[b] : Survivants à long terme au jour 50 sur le nombre de souris infectées et traitées.
[c] : Dose prophylactique causale guérissant 50% des animaux infectés.
[d] : L'index Pq est le rapport entre la $CPD_{50}$ de la Pq et la $CPD_{50}$ du dérivé.

Tableau 7 : Activité prophylactique causale de la L-glutamyl-primaquine et de la L-glutaminyl-primaquine

| Dérivés quinoléiniques[a] | Dose (mg/kg) | LTS/N[b] | LTS (%) | $CPD_{50}$[c] | Index[d] Pq |
|---|---|---|---|---|---|
| Contrôles | - | 0/50 | 0 | - | - |
| | 4,2 | 2/15 | 13 | | |
| | 6,2 | 1/5 | 20 | | |
| | 7,2 | 2/5 | 40 | | |
| L-glu-Pq | 8,1 | 9/20 | 45 | 8,5 | 2,2 |
| | 10,0 | 11/20 | 55 | | |
| | 12,2 | 10/15 | 67 | | |
| | 14,8 | 10/10 | 100 | | |
| | 16,0 | 5/5 | 100 | | |
| | 2,5 | 0/10 | 0 | | |
| | 5,0 | 1/10 | 10 | | |
| | 7,7 | 7/25 | 28 | | |
| L-gln-Pq | 10,1 | 5/10 | 50 | 10,0 | 1,9 |
| | 11,7 | 12/20 | 60 | | |
| | 15,6 | 12/15 | 80 | | |
| | 20,2 | 13/14 | 93 | | |

a : Les deux dérivés de la Primaquine sont administrés par voie i.v. 3 heures après l'inoculation des sporozoïtes. Les concentrations sont également exprimées en équivalent Pq diphosphate/kg.

b : Survivants à long terme au jour 50 sur le nombre de souris infectées et traitées.

c : Dose prophylactique causale guérissant 50% des animaux infectés.

d : L'index pq est le rapport entre la $CPD_{50}$ de la Pq et la $CPD_{50}$ du dérivé.

Tableau 8 : Activité prophylactique causale de la L-leucyl-L-alanyl-primaquine

| Dérivés quinoléiniques[a] | Dose (mg/kg) | LTS/N[b] | LTS (%) | $CPD_{50}$[c] | Index[d] Pq |
|---|---|---|---|---|---|
| Contrôles | - | 0/25 | 0 | - | - |
| L-leu-L-ala-Pq | 4,8 | 1/10 | 10 | | |
| | 9,5 | 2/10 | 20 | 13,9 | 1,35 |
| | 13,9 | 8/16 | 50 | | |
| | 18,6 | 8/15 | 53 | | |
| | 23,5 | 10/10 | 100 | | |

a : Le dérive de la Primaquine est administré par voie i.v. 3 heures après l'inoculation des sporozoïtes. Les concentrations sont également exprimées en équivalent Pq diphosphate/kg.
b : Survivants à long terme au jour 50 sur le nombre de souris traités.
c : Dose prophylactique causale guérissant 50% des animaux infectés.
d : L'index Pq est le rapport entre la $CPD_{50}$ de la Pq et |a $CPD_{50}$ du dérivé.

On a également étudié l'effet résiduel éventuel de tous ces composés sur les formes sanguines. Aucun des composés ne montre une activité inhibitrice des premières formes sanguines (mérozoïtes hépatiques) à une concentration correspondant au $CPD_{50}$ et $ED_{100}$. Dès lors, l'effet thérapeutique obtenu provient bien de la destruction des formes hépatiques (exoérythrocytaires) et représente une activité prophylactique causale vraie.

Il apparaît clairement des résultats obtenus dans le modèle de la malaria, que l'addition d'un acide aminé sur la chaîne latérale de la primaquine par l'intermédiaire d'un lien peptidique permet, d'une part d'accroître l'activité thérapeutique de la primaquine et, d'autre part, de réduire sensiblement sa toxicité aiguë.

Sur base de l'index Pq, les meilleures activités thérapeutiques sont obtenues pour la L-lysyl-Pq (4,8), la L-phénylalanyl-Pq (3,1), la L-alanyl-Pq (3,0) et la L-glutamyl-Pq (2,2). Les autres dérivés de la primaquine présentent une activité thérapeutique similaire à celle de la primaquine.

Sur base de la toxicité aiguë de la primaquine et de ses dérivés, la meilleure réduction de toxicité est obtenue pour la L-glutamyl-Pq, la L-alanyl-Pq et la L-leucyl-L-alanyl-Pq. La L-lysyl-Pq quant à elle se montre nettement plus toxique que la primaquine (3x).

Les deux effets combinés (activité et toxicité) permettent une augmentation appréciable de l'index thérapeutique particulièrement dans le cas du dérivé L-glutamyl-Pq (16,1), L-alanyl-Pq (7,5) et L-phénylalanyl-Pq (5,3).

L'addition d'un second acide aminé à la primaquine conduit à une augmentation de l'index thérapeutique notamment pour le dérivé L-leucyl-L-alanyl-Pq (3,7).

**Revendications**

**Revendications pour les Etats contractants suivants : AT, BE, CH, DE, FR, GB, IT, LI, LU, NL, SE**

1. Dérivés de quinoléine ayant des propriétés anti-malariques, ou leurs sels d'addition avec des acides, caractérisés en ce qu'ils sont représentés schématiquement par la formule :

PQ - X

dans laquelle,

- PQ est une 8-aminoquinoléine antimalarique et X est choisi parmi la L-alanine, la L-phénylalanine, l'acide L-glutamique.
- la liaison PQ-X étant une liaison covalente peptidique entre le groupement amine libre de PQ et le groupement carboxylique terminal de X.

**2.** Dérivés selon la revendication 1, caractérisés en ce que PQ est la primaquine.

**3.** Procédé de préparation des dérivés selon la revendication 1, caractérisé en ce qu'on fait réagir par condensation X, dont la fonction amine est protégée, avec PQ ou un de ses sels, portant sa fonction amine libre, en présence d'un agent de condensation pour former la liaison peptidique PQ-X.

**4.** Procédé selon la revendication 3, caractérisé en ce que le groupement protecteur de la fonction amine est un groupement tertiobutyloxycarbonyle.

**5.** Procédé selon la revendication 3 ou 4 caractérisé en ce que la fonction acide de l'aminoacide est activée sous forme d'ester avec le N-hydroxysuccinimide.

**6.** Procédé selon la revendication 3 caractérisé en ce qu'on part du diphosphate de primaquine, et de l'acide aminé (X), dont la fonction aminée est protégée par un groupe terbutyloxycarbonyle, et on effectue les étapes suivantes :

a) on fait réagir de la N-hydroxysuccinimide sur le dérivé N-terbutyloxycarbonyle de X.

b) on fait réagir PQ libérée à partir de son sel par une solution d'ammoniaque, avec l'ester N-hydroxysuccinimide du dérivé protégé de X,

c) le produit brut ainsi obtenu est purifié par chromatographie sur silicagel,

d) le groupement protecteur, terbutyloxy-carbonyle du composé obtenu, est ensuite clivé en présence d'acide trifluoroacétique pour donner le dérivé PQ-X sous forme de sel trifluoroacétate.

**7.** Procédé selon la revendication 6, caractérisé en ce que le produit obtenu après l'étape d) est purifié par chromatographie en phase inverse.

**8.** Procédé selon la revendication 7, caractérisé en ce qu'on utilise comme éluant un mélange d'acétonitrile dans l'eau contenant de l'acide trifluoroacétique.

**9.** Procédé selon la revendication 6, caractérisé en ce que le contre anion trifluoroacétate est échangé en chlorhydrate.

**10.** Procédé selon la revendication 9, caractérisé en ce que le sel chlorhydrate est traité par du bisulfite de sodium.

**11.** Composition pharmaceutique contenant à titre de principe actif les nouveaux dérivés selon la revendication 1 ou leurs sels pharmaceutiquement acceptables.

**Revendications pour les Etats contractants suivants : ES, GR**

**1.** Procédé de préparation de dérivés de quinoléine ayant des propriétés anti-malariques, ou leurs sels d'addition avec des acides, représentés schématiquement par la formule :

PQ - X

dans laquelle,

- PQ est une 8-aminoquinoléine antimalarique et X est choisi parmi la L-alanine, la L-phénylalamine, l'acide L-glutamique, dans lequel on effectue un couplage covalent, la liaison PQ-X étant une liaison covalente peptidique entre le groupement amine libre de PQ et le groupement carboxylique terminal de X.

**2.** Procédé selon la revendication 1, caractérisé en ce que PQ est la primaquine.

**3.** Procédé de préparation des dérivés selon la revendication 1, caractérisé en ce qu'on fait réagir par condensation X, dont la fonction amine est protégée, avec PQ ou un de ses sels, portant sa fonction amine libre, en présence d'un agent de condensation pour former la liaison peptidique PQ-X.

**4.** Procédé selon la revendication 3, caractérisé en ce que le groupement protecteur de la fonction amine est un groupement tertiobutyloxycarbonyle.

**5.** Procédé selon la revendication 3 ou 4 caractérisé en ce que la fonction acide de l'aminoacide est activée sous forme d'ester avec le N-hydroxysuccinimide.

**6.** Procédé selon la revendication 3, caractérisé en ce qu'on part du diphosphate de primaquine, et de l'acide aminé (X), dont la fonction aminée est protégée par un groupe terbutyloxycarbonyle, et on effectue les étapes suivantes :
   a) on fait réagir de la N-hydroxysuccinimide sur le dérivé N-terbutyloxycarbonyle de X ;
   b) on fait réagir PQ libérée à partir de son sel par une solution d'ammoniaque, avec l'ester N-hydroxysuccinimide du dérivé protégé de
   c) le produit brut ainsi obtenu est purifié par chromatographie sur silicagel;
   d) le groupement protecteur, terbutyloxy-carbonyle du composé obtenu, est ensuite clivé en présence d'acide trifluoroacétique pour donner le dérivé PQ-X sous forme de sel trifluoroacétate.

**7.** Procédé selon la revendication 6, caractérisé en ce que le produit obtenu après l'étape d) est purifié par chromatographie en phase inverse.

**8.** Procédé selon la revendication 7, caractérisé en ce qu'on utilise comme éluant un mélange d'acétonitrile dans l'eau contenant de l'acide trifluoroacétique.

**9.** Procédé selon la revendication 6, caractérisé en ce que le contre anion trifluoroacétate est échangé en chlorydrate.

**10.** Procédé selon la revendication 9, caractérisé en ce que le sel chlorhydrate est traité par du bisulfite de sodium.

**11.** Composition pharmaceutique contenant à titre de principe actif les nouveaux dérivés selon la revendication 1 ou leurs sels pharmaceutiquement acceptables.

**Claims**
**Claims for the following Contracting States : AT, BE, CH, DE, FR, GB, IT, LI, LU, NL, SE**

**1.** Quinoline derivatives having antimalarial properties or their their addition salts with acids, which are represented schematically by the formula:

PQ - X

in which
- PQ is an antimalarial 8-aminoquinoline,
- X is selected from L-alanine, L-phenylalanine, L-glutamic acid,
- the PQ - X bond being a covalent peptide bond between the free amine group of PQ and the terminal carboxyl group of X.

**2.** The derivatives as claimed in claim 1, in which PQ is primaquine.

**3.** A process for preparing new derivatives as claimed in claim 1, wherein X, in which the amine group is optionally protected, is reacted by condensation with PQ or one of its salts, bearing its free amine group, in the presence of a condensing agent to form the PQ - X peptide bond.

**4.** The process as claimed in claim 3, wherein the group protecting the amine group is a tert-butyloxycarbonyl group.

**5.** The process as claimed in claim 3 or 4, wherein the acid group of the amino acid is activated in the form of the ester with N-hydroxysuccinimide.

**6.** The process as claimed in claim 3, wherein the starting material is primaquine diphosphate, and the amino acid (X) in which the amino group is protected, for example by a tert-butyloxycarbonyl group, and the following stages are performed:
   a) N-hydroxysuccinimide is reacted with the N-tert-butyl-oxycarbonyl derivative of X,
   b) PQ, liberated from its salt with amonia solution is reacted; it is reacted with the N-hydroxysuccinimide ester of the protected derivative of X,
   c) the crude product thereby obtained is purified by chromatography on silica gel,
   d) the tert-butyloxycarbonyl protective group on the compound obtained is then cleaved in the presence of trifluoroacetic acid, to give the derivative PQ - X in trifluoroacetate salt form.

**7.** The process as claimed in claim 6, wherein the product obtained after stage d) is purified by reverse phase chromatography.

**8.** The process as claimed in claim 7, wherein a mixture of acetonitrile in water containing trifluoroacetic acid, is used as eluant.

**9.** The process as claimed in claim 6, wherein the trifluoroacetate counter-anion is exchanged for hydrochloride.

**10.** The process as claimed in claim 9, wherein the hydrochloride salt is treated with sodium bisulfite.

**11.** A pharmaceutical composition containing , by way of active principle, the new derivatives as claimed in claim 1 or their pharmaceutically acceptable salts.

**Claims for the following Contracting States : ES, GR**

**1.** Process for the preparation of quinoline derivatives having antimalarial properties or their addition salts with acids, which are represented schematically by the formula:

PQ - X

in which
- PQ is an antimalarial 8-aminoquinoline,
- X is selected from L-alanine, L-phenylalanine, L-glutamic acid wherein a covalent linkage is performed,

the PQ - X bond being a covalent peptide bond between the free amine group of PQ and the terminal carboxyl group of X.

**2.** The process as claimed in claim 1, in which PQ is primaquine.

**3.** A process for preparing new derivatives as claimed in claim 1, wherein X, in which the amine groups are optionally protected, is reacted by condensation with PQ or one of its salts, bearing its free amine group, in the presence of a condensing agent to form the PQ - X peptide bond.

**4.** The process as claimed in claim 3, wherein the group protecting the amine group is a tert-butyloxycarbonyl group.

**5.** The process as claimed in claim 3 or 4, wherein the acid group of the amino acid is activated in the form of the ester with N-hydroxysuccinimide.

**6.** The process as claimed in claim 3, wherein the starting material is primaquine diphosphate, and the amino acid (X) in which the amino group is protected, for example by a tert-butyloxycarbonyl group, and the following stages are performed:
   a) N-hydroxysuccinimide is reacted with the N-tert-butyl-oxycarbonyl derivative of X,
   b) PQ, liberated from its salt with amonia solution is reacted; it is reacted with the N-hydroxysuccinimide ester of the protected derivative of X,
c) the crude product thereby obtained is purified by chromatography on silica gel,
d) the tert-butyloxycarbonyl protective group on the compound obtained is then cleaved in the presence of trifluoroacetic acid, to give the derivative PQ - X in trifluoroacetate salt form.

**7.** The process as claimed in claim 6, wherein the product obtained after stage d) is purified by reverse phase chromatography.

**8.** The process as claimed in claim 7, wherein a mixture of acetonitrile in water containing trifluoroacetic acid, is used as eluant.

**9.** The process as claimed in claim 6, wherein the trifluoroacetate counter-anion is exchanged for hydrochloride.

**10.** The process as claimed in claim 9, wherein the hydrochloride salt is treated with sodium bisulfite.

**11.** A pharmaceutical composition containing , by way of active principle, the new derivatives as claimed in claim 1 or their pharmaceutically acceptable salts.

## Patentansprüche

**Patentansprüche für folgende Vertragsstaaten : AT, BE, CH, DE, FR, GB, IT, LI, LU, NL, SE**

**1.** Chinolin-Derivate mit Anti-Malaria-Eigenschaften, oder ihre Additionssalze mit Säuren, dadurch gekennzeichnet, daß sie schematisch der allgemeinen Formel

PQ - X

entsprechen, in der
- PQ ein Antimalaria-8-Aminochinolin darstellt und X ausgewählt wird unter L-Alanin, L-Phenylalanin und L-Glutaminsäure,

wobei die Bindung PQ-X eine kovalente Peptid-Bindung zwischen der freien Amino-Gruppe des PQ und der endständigen Carbonsäure-Gruppe des X ist.

**2.** Derivate nach Anspruch 1, dadurch gekennzeichnet, daß PQ Primaquin ist.

**3.** Verfahren zur Herstellung der Derivate nach Anspruch 1, dadurch gekennzeichnet, daß man X, dessen Amin-Funktion geschützt ist, mit PQ oder einem seiner Salze, das die freie Amin-Funktion trägt, in Anwesenheit eines Kondensationsmittels, durch Kondensation zur Reaktion bringt, um die Peptid-Bindung PQ-X zu bilden.

**4.** Verfahren nach Anspruch 3, dadurch gekennzeichnet, daß die Schutzgruppe der Amin-Funktion eine tert.-Butyloxycarbonyl-Gruppe ist.

**5.** Verfahren nach Anspruch 3 oder 4, dadurch gekennzeichnet, daß die Aminosäure-Funktion in Form des Esters mit N-Hydroxysuccinimid aktiviert wird.

**6.** Verfahren nach Anspruch 3, dadurch gekennzeichnet, daß man vom Primaquin-Diphosphat und der Aminosäure (X) ausgeht, deren Amin-Funktion durch eine Tert.-Butyloxycarbonyl-Gruppe geschützt ist, und man die folgenden Stufen durchführt:
a) Reaktion des N-Hydroxysuccinimids mit dem N-tert.-Butyloxycarbonyl-Derivatvon X,
b) Reaktion des ausgehend von seinem Salz durch eine Ammoniak-Lösung freigesetzten PQ mit dem N-Hydroxysuccinimid-Ester des geschützten Derivates X,
c) Reinigung des auf diese Weise erhaltenen rohen Produktes durch Chromatographie auf Silicagel,
d) anschließende Abspaltung der Schutzgruppe tert.-Butyloxycarbonyl von der erhaltenen Verbindung in Anwesenheit von Trifluoressigsäure, um das Derivat PQ-X in Form seines Trifluoracetates zu erhalten.

**7.** Verfahren nach Anspruch 6, dadurch gekennzeichnet, daß das nach der Stufe d) erhaltene Produkt

durch Umkehrphasen-Chromatographie gereinigt wird.

**8.** Verfahren nach Anspruch 7, dadurch gekennzeichnet, daß man als Eluent eine Mischung von Acetonitril in Wasser verwendet, die Trifluoressigsäure enthält.

**9.** Verfahren nach Anspruch 6, dadurch gekennzeichnet, daß das Gegenanion Trifluoracetat durch Hydrochlorid ausgetauscht wird.

**10.** Verfahren nach Anspruch 9, dadurch gekennzeichnet, daß das Hydrochlorid-Salz mit Natriumbisulfit behandelt wird.

**11.** Pharmazeutische Zusammensetzung, enthaltend als Wirkstoff die neuen Derivate nach Anspruch 1 oder ihre pharmazeutisch akzeptablen Salze.

**Patentansprüche für folgende Vertragsstaaten : ES, GR**

**1.** Verfahren zur Herstellung von Chinolin-Derivaten mit Anti-Malaria-Eigenschaften, oder ihren Additionssalzen mit Säuren, die schematisch der allgemeinen Formel

PQ - X

entsprechen, in der

- PQ ein Antimalaria-8-Aminochinolin darstellt und X ausgewählt wird unter L-Alanin, L-Phenylalanin und L-Glutaminsäure, bei dem man eine kovalente Kupplung durchführt, wobei die Bindung PQ-X eine kovalente Peptid-Bindung zwischen der freien Amino-Gruppe des PQ und der endständigen Carbonsäure-Gruppe des X ist.

**2.** Verfahren nach Anspruch 1, dadurch gekennzeichnet, daß PQ Primaquin ist.

**3.** Verfahren zur Herstellung der Derivate nach Anspruch 1, dadurch gekennzeichnet, daß man X, dessen Amin-Funktion geschützt ist, mit PQ oder einem seiner Salze, das die freie Amin-Funktion trägt, in Anwesenheit eines Kondensationsmittels, durch Kondensation zur Reaktion bringt, um die Peptid-Bindung PQ-X zu bilden.

**4.** Verfahren nach Anspruch 3, dadurch gekennzeichnet, daß die Schutzgruppe der Amin-Funktion eine tert.-Butyloxycarbonyl-Gruppe ist.

**5.** Verfahren nach Anspruch 3 oder 4, dadurch gekennzeichnet, daß die Aminosäure-Funktion in Form des Esters mit N-Hydroxysuccinimid aktiviert wird.

**6.** Verfahren nach Anspruch 3, dadurch gekennzeichnet, daß man vom Primaquin-Diphosphat und der Aminosäure (X) ausgeht, deren Amin-Funktion durch eine Tert.-Butyloxycarbonyl-Gruppe geschützt ist, und man die folgenden Stufen durchführt:

a) Reaktion des N-Hydroxysuccinimids mit dem N-tert.-Butyloxycarbonyl-Derivatvon X,

b) Reaktion des ausgehend von seinem Salz durch eine Ammoniak-Lösung freigesetzten PQ mit dem N-Hydroxysuccinimid-Ester des geschützten Derivates X,

c) Reinigung des auf diese Weise erhaltenen rohen Produktes durch Chromatographie auf Silicagel,

d) anschließende Abspaltung der Schutzgruppe tert.-Butyloxycarbonyl von der erhaltenen Verbindung in Anwesenheit von Trifluoressigsäure, um das Derivat PQ-X in Form seines Trifluoracetates zu erhalten.

**7.** Verfahren nach Anspruch 6, dadurch gekennzeichnet, daß das nach der Stufe d) erhaltene Produkt durch Umkehrphasen-Chromatographie gereinigt wird.

**8.** Verfahren nach Anspruch 7, dadurch gekennzeichnet, daß man als Eluent eine Mischung von Acetonitril in Wasser verwendet, die Trifluoressigsäure enthält.

**9.** Verfahren nach Anspruch 6, dadurch gekennzeichnet, daß das Gegenanion Trifluoracetat durch Hydrochlorid ausgetauscht wird.

**10.** Verfahren nach Anspruch 9, dadurch gekennzeichnet, daß das Hydrochlorid-Salz mit Natriumbisulfit behandelt wird.

**11.** Pharmazeutische Zusammensetzung, enthaltend als Wirkstoff die neuen Derivate nach Anspruch 1 oder ihre pharmazeutisch akzeptablen Salze.